(19) Europäisches Patentamt
European Patent Office
Office européen des brevets

(11) EP 3 331 513 B1

(12) **FASCICULE DE BREVET EUROPEEN**

(45) Date de publication et mention de la délivrance du brevet:
**17.03.2021 Bulletin 2021/11**

(21) Numéro de dépôt: **16760132.7**

(22) Date de dépôt: **05.08.2016**

(51) Int Cl.:
***A61K 31/155*** (2006.01) ***A61K 31/352*** (2006.01)
***A61P 31/02*** (2006.01) ***A61L 2/18*** (2006.01)

(86) Numéro de dépôt international:
**PCT/FR2016/052037**

(87) Numéro de publication internationale:
**WO 2017/021667 (09.02.2017 Gazette 2017/06)**

# (54) SOLUTIONS HYDROALCOOLIQUES COLOREES DE CHLORHEXIDINE, LEURS PROCEDES DE FABRICATION ET LEURS UTILISATIONS

MIT CHLORHEXIDIN GEFÄRBTE HYDROALKOHOLISCHE LÖSUNGEN, VERFAHREN ZUR HERSTELLUNG DAVON UND VERWENDUNGEN DAVON

HYDROALCOHOLIC SOLUTIONS COLOURED WITH CHLORHEXIDINE, METHODS FOR THE PRODUCTION THEREOF, AND USES OF SAME

(84) Etats contractants désignés:
**AL AT BE BG CH CY CZ DE DK EE ES FI FR GB GR HR HU IE IS IT LI LT LU LV MC MK MT NL NO PL PT RO RS SE SI SK SM TR**

(30) Priorité: **06.08.2015 FR 1557594**

(43) Date de publication de la demande:
**13.06.2018 Bulletin 2018/24**

(73) Titulaires:
- **Université Claude Bernard Lyon I**
  **69622 Villeurbanne Cedex (FR)**
- **Hospices Civils De Lyon**
  **69229 Lyon Cedex 02 (FR)**

(72) Inventeurs:
- **PIROT, Fabrice**
  **69006 Lyon (france) (FR)**
- **FILALI, Samira**
  **69003 Lyon (FR)**
- **LAMINE TALL, Mamadou Lamine**
  **93300 Aubervilliers (FR)**
- **PIVOT, Christine**
  **69200 Venissieux (FR)**

(74) Mandataire: **Be IP**
**Cabinet LTL SAS**
**Centre d'Entreprise et d'Innovation**
**56, Bd Niels Bohr**
**CS 52132**
**69603 Villeurbanne Cedex (FR)**

(56) Documents cités:
**EP-A1- 2 499 913**
**US-A1- 2003 059 379**
**US-A1- 2008 081 020**

EP 3 331 513 B1

Il est rappelé que: Dans un délai de neuf mois à compter de la publication de la mention de la délivrance du brevet européen au Bulletin européen des brevets, toute personne peut faire opposition à ce brevet auprès de l'Office européen des brevets, conformément au règlement d'exécution. L'opposition n'est réputée formée qu'après le paiement de la taxe d'opposition. (Art. 99(1) Convention sur le brevet européen).

## Description

Le domaine de l'invention est celui des compositions antiseptiques. L'invention concerne, en particulier, des compositions colorées et stables de chlorhexidine, leurs procédés de fabrication et leurs utilisations pour l'antisepsie de la peau ou des instruments, notamment en vue d'une intervention chirurgicale.

La chlorhexidine (1,6-di(4'-chlorophényldiguanido)hexane) est un antiseptique à large spectre d'action qui possède des propriétés bactéricides. La chlorhexidine est utilisée en médecine pour la désinfection des instruments, des mains et de la peau, notamment avant une intervention chirurgicale. Elle est aussi utilisée en orthodontie pour le soin des dents (US 6,337,357, notamment). La chlorhexidine est une base (pKa =10,8) utilisée le plus souvent sous la forme d'un sel cationique pour des questions de solubilité dans l'eau.

Lorsque la chlorhexidine ou l'un de ses sels est dissout dans une solution aqueuse ou une solution hydroalcoolique, la solution est incolore. Or, la chlorhexidine est souvent utilisée pour désinfecter et pour l'antisepsie de la peau ou des instruments avant une intervention chirurgicale. Du fait qu'elle se présente sous la forme d'une solution incolore, il est difficile, d'une part, de vérifier si la solution a été réellement appliquée sur la peau ou sur les instruments et, d'autre part, de délimiter la zone de la peau ou de l'instrument qui a été effectivement désinfectée. Il en résulte que les vérifications préopératoires sont difficiles à effectuer et qu'un chirurgien ne peut pas visualiser rapidement la zone sur laquelle il doit intervenir et vérifier si celle-ci a bien été soumise à une désinfection.

Pour pallier ces problèmes, plusieurs solutions colorées de chlorhexidine ont été développées. Les colorants utilisés sont ajoutés directement dans les solutions de chlorhexidine ou extemporanément au moment de l'application.

On peut, par exemple, citer la solution antiseptique d écrite dans le document EP 2 499 913 comprenant a) au moins un dérivé di(4-chlorophenyldiguanido) ou un sel pharmaceutiquement acceptable et b) au moins un colorant anionique. Un autre exemple est la solution commerciale Chloraprep® comprenant 70/30 v/v d'isopropanol/eau, du jaune orangé S (colorant E110) et du digluconate de chlorhexidine 2 % m/v par rapport au volume total de la composition. Le jaune orangé S est un colorant anionique. Le ratio molaire de digluconate de chlorhexidine/jaune orangé S est égal à 2,7. Cette solution permet une coloration de la peau en jaune orange. Toutefois, cette coloration est faible. En outre, comme cette solution n'est pas stable dans le temps, elle est utilisée avec un applicateur à usage unique. Ce dernier permet le mélange du colorant et du sel de chlorhexidine extemporanément au moment de désinfecter la peau avant une intervention médicale invasive par exemple.

Mölnlycke Health Care® propose une solution moussante (appelée Hibiscrub® 4%) de digluconate de chlorhexidine 4% dans l'isopropanol colorée par du rouge cochenille A (colorant E124). Cette solution est destinée au traitement local d'appoint et au nettoyage des affections de la peau infectée ou risquant de s'infecter, à l'antisepsie des mains du personnel soignant et à l'antisepsie chirurgicale. Le ratio molaire de digluconate de chlorhexidine/rouge cochenille A est égal à 438. Cette solution est prête à l'emploi, mais ne permet pas d'obtenir une coloration de la peau qui soit perceptible. En outre, le colorant rouge cochenille A est interdit aux USA car il serait potentiellement cancérigène.

Le document US 2008/0081020, quant à lui, décrit une préparation chirurgicale, sous la forme d'une solution, pouvant ou non former un film, qui change de couleur quand le pH de la solution change, pour indiquer que le solvant s'est suffisamment évaporé. Cette solution est complexe et inclut un agent antimicrobien, un solvant, un ajusteur de pH, un indicateur de changement de couleur et optionnellement, un polymère formant un film, un colorant et un régulateur de viscosité. De nombreux exemples de colorant, parmi lesquels figure le Red #22, sont donnés et la chlorhexidine digluconate est citée parmi d'autres agents antimicrobien. Aucun des exemples n'utilise le Red #22 en tant qu'agent colorant. De surcroit, le procédé décrit à l'exemple B prévoit de mélanger tous les constituants en même temps, ce qui conduit à une composition instable si du Red #22 est utilisé. L'exemple B utilise un rapport molaire digluconate de chlorhexidine/FD&C Red 40 de 129 ou digluconate de chlorhexidine/bromothymol Blue de 8137 et l'exemple C un rapport molaire digluconate de chlorhexidine/bromothymol Blue de 5374.

On peut encore citer le document US 2003/0059379 qui décrit des méthodes pour traiter des maladies parodontales, ou d'autres maladies des tissus, qui utilisent une composition colorée et un traitement sous énergie laser. La composition colorée comprend un colorant qui absorbe de la lumière à une énergie comprise entre 450 et 600 nanomètres. Le Red #28 est donné en tant qu'exemple de colorants parmi d'autres. Il est également prévu que cette composition comprenne un antibiotique qui peut être de la chlorhexidine gluconate, citée parmi d'autres exemples d'antibiotique. Là encore, aucune composition comprenant en association de la chlorhexidine gluconate et un tel colorant n'est décrite dans ce document. Par ailleurs, dans ce document, la proportion de colorant est supérieure à celle de la chlorhexidine, avec des ratios [digluconate de chlorhexidine]/[colorant] de 0,13 - 0,09 et 0,13 respectivement aux exemples 1, 3 et 4. Par ailleurs, les compositions décrites dans ce document comprennent une faible proportion d'éthanol, ce qui joue sur leur stabilité.

Les laboratoires Gilbert® ont proposé une solution alcoolique de chlorhexidine colorée pour l'antisepsie du champ opératoire. Le colorant utilisé était le D&C Red 27 de formule :

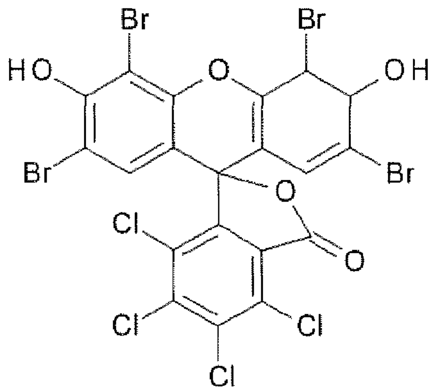

Le ratio molaire de digluconate de chlorhexidine/D&C Red 27 dans la solution alcoolique proposée était égal à 13. Cette solution prête à l'emploi, était utilisée en badigeonnage du champ opératoire et permettait une coloration rouge orangé faible de la peau. Du fait de problèmes d'instabilité observés, cette solution a cessé d'être commercialisée.

Le document FR 2791890 indique, quant à lui, que la couleur rouge doit être évitée (voir page 2 lignes 19-20) et propose une composition, pour une application cutanée en vue de l'antisepsie du champ opératoire, comprenant du digluconate de chlorhexidine, de l'éthanol, et un mélange de deux colorants : du jaune de quinoléine et du bleu patenté. Le ratio molaire de digluconate de chlorhexidine/jaune de quinoléine est égal à 6 et le ratio molaire de digluconate de chlorhexidine/bleu patenté est égal à 73.

Cette composition est obtenue par mélange des solutions de colorants, préparées et filtrées moins de 48 heures avant leur mélange à la solution de digluconate de chlorhexidine. Après mélange, la composition finale est à nouveau filtrée. Il est indiqué que la filtration retient une certaine quantité de colorant sans que cela affecte le pouvoir de coloration. La stabilité de cette solution a été testée. Cette solution est stable jusqu'à trois mois. Toutefois, au-delà de quatre mois de stockage, on observe un précipité. L'effet bactéricide de cette composition n'est pas présenté dans le document FR 2791890. Cette solution présente, en outre, l'inconvénient de marquer la peau en vert, couleur qui est difficilement visible sous les lampes utilisées dans le bloc opératoire, et notamment sous scialytique. Par ailleurs, l'utilisation du jaune de quinoléine ou du bleu patenté peut provoquer des effets secondaires tels que des allergies, des rhinites ou de l'urticaire.

Par ailleurs, la littérature scientifique rapporte des publications montrant une diminution de l'efficacité de la chlorhexidine en association avec un colorant (BLECH, M. F., MARTIN, C., PICHON, M., et al., Clinical and bacteriologic course of wounds as a function of various protocols of local antisepsis, Revue de chirurgie orthopédique et réparatrice de l'appareil moteur, 1989, vol. 76, no 1, p. 55-61 ; CHOW, Jason, NG, Jonathan, et PUN, Alvin, Effects of food colouring added to 2% chlorhexidine gluconate and 70% alcohol for surgical site antisepsis, Journal of perioperative practice, 2013, vol. 23, no 11, p. 255-257). J. CHOW et al. notamment décrivent que l'efficacité de la chlorhexidine est diminuée lorsqu'elle est utilisée en mélange avec un colorant alimentaire, les colorants alimentaires étant présentés comme généralement anioniques.

Il apparaît donc qu'il est difficile d'obtenir une solution de chlorhexidine ou d'un de ses sels qui soit stable dans le temps, c'est-à-dire qui ait une durée d'utilisation longue sans formation de précipités incompatibles avec une administration cutanée, et qui permette une coloration de la peau qui soit visible notamment au bloc opératoire, tout en bénéficiant des propriétés antiseptiques de la chlorhexidine.

Pour pallier au problème de stabilité et de précipitation ou perte d'activité constatée de la chlorhexidine en présence de différents colorants anioniques, il a été proposé d'associer ces colorants anioniques à des composés organiques cationiques. En effet, le document WO 2007/130981 a essayé de résoudre le problème précité en proposant une solution aqueuse de chlorhexidine colorée avec des colorants anioniques en combinaison avec un excipient cationique ou un colorant cationique. Les colorants anioniques envisagés dans cette demande de brevet sont le FD&C Blue N°1 (Brillant blue FCF), FD&C Blue N°2 (Indigo Carmine), FD&C Green N°3 (Fast Green FCF), FD&C Red N°3 (Erythrosine), FD&C Red N°40 (Allura Red), FD&C Yellow N°5 (Tartrazine), et FD&C Yellow N°6 (Sunset yellow FCF). Il est décrit dans ce document que les colorants anioniques, notamment ceux mentionnés ci-dessus, forment un précipité avec la chlorhexidine ou avec ses sels, même lorsque ces colorants sont utilisés à de très faibles concentrations. En outre, il est mentionné que l'utilisation seule d'un colorant anionique à une solution aqueuse de chlorhexidine diminue l'efficacité de la solution, du fait de la formation d'un précipité entre le colorant anionique et la chlorhexidine ou ses sels. Lorsqu'un colorant ou un excipient cationique est présent, il masque la charge négative du colorant anionique et aucune formation de précipité n'est alors observée. Dans ce document, l'excipient ou le colorant cationique est donc un élément essentiel de la composition de chlorhexidine pour obtenir une composition colorée. De plus, les compositions décrites dans le document WO 2007/130981 présentent l'inconvénient d'être nocives pour la santé, car ces colorants cationiques utilisés provoquent des brûlures cutanées ou sont des composés CMR, c'est-à-dire Cancérigène, Mutagène et Reprotoxique.

Il subsiste donc toujours un besoin de disposer d'une composition de chlorhexidine qui pallie les inconvénients des compositions de l'art antérieur.

Un objectif de la présente invention est de disposer d'une composition colorée de chlorhexidine, qui rassemble les caractéristiques suivantes : présenter une bonne stabilité dans le temps, présenter une bonne efficacité antibactérienne, ne pas être dangereuse pour la santé des patients sur laquelle elle serait appliquée ou du personnel médical qui l'utiliserait, être apte à marquer la peau et fournir un marquage coloré visible notamment sous les lumières d'un bloc opératoire tel que le scialytique.

Dans ce contexte, l'invention propose des compositions liquides stables comprenant :

- du digluconate de chlorhexidine (n° CAS : 18472-51-0),
- un colorant de formule (I) sous la forme d'un sel pharmaceutiquement acceptable,

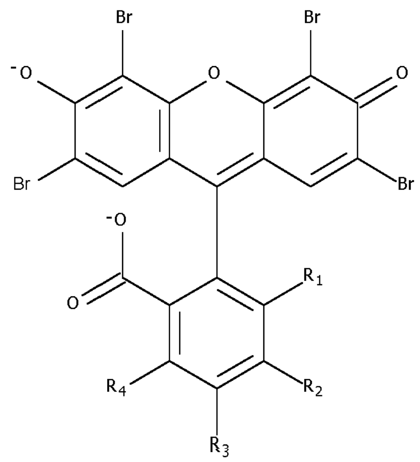

(I)

dans laquelle $R_1$, $R_2$, $R_3$, $R_4$, identiques ou différents, représentent chacun indépendamment un atome d'hydrogène ou un atome de chlore,

- de l'eau,
- et au moins un alcool choisi parmi l'éthanol, le n-propanol et l'isopropanol, ladite composition comprenant de 0,4 à 2,5% m/v, par rapport au volume total de la composition, de digluconate de chlorhexidine et de 0,01 à 0,06% m/v, par rapport au volume total de ma composition, d'une sel pharmaceutiquement acceptable du colorant de formule (I).

Le digluconate de chlorhexidine, est un antiseptique bactéricide efficace contre une large gamme de bactéries Gram positives et Gram négatives. Il possède une activité plus marquée contre les bactéries Gram positives. Ce composé a aussi une activité sur un certain nombre de champignons, de moisissures et de virus enveloppés. En revanche, il est inefficace contre les germes acido-résistants, les virus nus et les spores bactériennes.

Le digluconate de chlorhexidine, sel de chlorhexidine sélectionné dans les compositions selon l'invention, est miscible à l'eau et assez soluble dans les alcools primaires en C2 et C3 et notamment dans l'éthanol. Le digluconate de chlorhexidine est disponible dans le commerce.

L'alcool présent dans la composition, et en particulier l'éthanol, peut renforcer le pouvoir bactéricide du digluconate de chlorhexidine.

Les mélanges digluconate de chlorhexidine/alcool en C2 ou C3/eau, sont particulièrement efficaces pour l'antisepsie de la peau, car ils combinent sur la peau les propriétés de rapidité antimicrobienne de l'alcool (notamment de l'éthanol), avec les propriétés de persistance du digluconate de chlorhexidine. Cette combinaison permet d'élargir le spectre d'activité du digluconate de chlorhexidine, notamment d'élargir son activité aux mycobactéries, notamment lorsqu'on utilise de l'éthanol à 70° (c'est-à-dire un mélange eau/alcool comprenant 70% v/v en alcool et 30% v/v d'eau).

De manière surprenante, il a été possible, dans le cadre de l'invention, de formuler une composition hydroalcoolique colorée et stable de digluconate de chlorhexidine, en ajoutant au moins un colorant anionique spécifique répondant à la formule (I), ce qui n'affecte pas les propriétés anti-bactériennes de la chlorhexidine et ce, sans qu'il soit nécessaire d'ajouter dans la composition d'autres composés organiques cationiques, autres que la chlorhexidine.

Le composé répondant à la formule (I) qui se trouve sous la forme d'un sel pharmaceutiquement acceptable dans la solution hydroalcoolique selon l'invention est une substance capable de colorer ladite solution hydroalcoolique. Il confère ainsi à une solution initialement translucide une certaine couleur, et en particulier une couleur rouge. Lorsque

la composition de l'invention est appliquée sur la peau, le sel du composé de formule (I) présent permet de donner une teinte à la peau différente de sa teinte d'origine. Il est ainsi possible de distinguer visuellement, soit à la lumière naturelle, soit à l'aide d'une lampe fluorescente, soit sous scialytique, les zones de la peau sur lesquelles la composition a été appliquée.

De manière avantageuse, le ratio molaire [digluconate de chlorhexidine]/[colorant de formule (I) sous la forme d'un sel pharmaceutiquement acceptable] appartient à la gamme allant de 6 à 197, de préférence à la gamme allant de 7 à 79. Préférentiellement, le ratio molaire [digluconate de chlorhexidine]/[colorant de formule (I) sous la forme d'un sel pharmaceutiquement acceptable] appartient à la gamme allant de 7 à 33, et préférentiellement à la gamme allant de 7 à 17, ce qui permet d'optimiser notamment la stabilité de la composition, en évitant plus longtemps une complexation et/ou une précipitation du digluconate de chlorhexidine et du colorant anionique.

De préférence, les compositions selon l'invention comprennent de 0,5 à 2 % m/v de digluconate de chlorhexidine et de 0,02 à 0,05% m/v, les % m/v d'un sel pharmaceutiquement acceptable du colorant de formule (I), étant exprimés par rapport au volume total de la composition.

De manière avantageuse, dans le colorant de formule (I) contenu dans les compositions selon l'invention, $R_1$, $R_2$, $R_3$ et $R_4$ sont tous identiques et représentent un atome d'hydrogène. Dans ce cas, le composé de formule (I) correspond à l'éosine (n° CAS : 17372-87-1). Le composé de formule (I) dans laquelle $R_1$, $R_2$, $R_3$ et $R_4$ sont tous identiques et représentent un atome de chlore est le D&C red 28 et peut également être utilisé. L'éosine, notamment, présente l'avantage d'être un colorant rouge, également fluorescent ($\lambda_{excitation}$ = 525 nm et $\lambda_{émission}$ = 545 nm). L'utilisation d'une lampe UV, pour mettre en évidence sa fluorescence, permet notamment de détecter les zones cutanées ou de muqueuses, peu accessibles à l'examen.

De manière avantageuse, dans les compositions selon l'invention, le colorant de formule (I) se présente sous la forme de son sel disodique ou dipotassique et est, de préférence, l'éosine disodique (n° CAS : 17372-87-1).

Préférentiellement, les compositions selon l'invention comprennent de 0,4 à 2,5% m/v de digluconate de chlorhexidine et de 0,01 à 0,06% m/v d'éosine disodique, de préférence de 0,5 à 2 % m/v de digluconate de chlorhexidine et de 0,02 à 0,05% m/v d'éosine disodique, les % m/v étant exprimés par rapport au volume total de la composition.

Préférentiellement, les compositions selon l'invention comprennent de 5 à 50 % v/v d'eau et de 50 à 95 % v/v d'alcool choisi parmi l'éthanol, le n-propanol et l'isopropanol et leurs mélanges, de préférence de 20 à 50 % v/v d'eau et de 50 à 80 % v/v d'alcool choisi parmi l'éthanol, le n-propanol et l'isopropanol et leurs mélanges, les % v/v étant exprimés par rapport au volume total de la composition.

De préférence, dans les compositions de l'invention, on utilisera un mélange de 20 à 40% v/v d'eau et de 60 à 80% v/v d'alcool, et en particulier d'éthanol, de préférence, un mélange de 25 à 35% v/v d'eau et de 65 à 75% v/v d'alcool, et en particulier d'éthanol, et de manière encore plus préférée, un mélange de 30% v/v d'eau et de 70% v/v d'alcool, et en particulier d'éthanol, ce qui permet d'optimiser encore les propriétés bactéricides et fongicides, l'activité sur les mycobactéries, l'activité virucide de la composition, celle-ci restant non sporicide.

A titre d'exemples préférés de composition selon l'invention, on peut citer les compositions liquides qui comprennent, voire qui sont exclusivement constituées de :

- de 0,4 à 2,5% m/v de digluconate de chlorhexidine, de préférence de 0,5 à 2 % m/v,
- de 0,01 à 0,06% m/v d'un sel pharmaceutiquement acceptable du colorant de formule (I), de préférence de 0,02 à 0,05% m/v,
- de 50 à 95 % v/v d'alcool choisi parmi l'éthanol, le n-propanol et l'isopropanol et leurs mélanges, de préférence de 50 à 80 % v/v et
- de 5 à 50 % v/v d'eau, de préférence de 20 à 50 % v/v.

A titre d'exemples particulièrement préférés de compositions selon l'invention, on peut citer les compositions liquides qui comprennent, voire qui sont exclusivement constituées de :

- de 0,4 à 2,5% m/v de digluconate de chlorhexidine, de préférence de 0,5 à 2 % m/v,
- de 0,01 à 0,06% m/v d'éosine disodique, de préférence de 0,02 à 0,05% m/v,
- de 50 à 95 % v/v d'alcool choisi parmi l'éthanol, le n-propanol et l'isopropanol et leurs mélanges, de préférence de 50 à 80 % v/v et
- de 5 à 50 % v/v d'eau, de préférence de 20 à 50 % v/v.

A titre d'exemples tout particulièrement préférés de compositions selon l'invention, on peut citer les compositions liquides qui comprennent, voire qui sont exclusivement constituées de :

- de 0,5 à 2% m/v de digluconate de chlorhexidine,
- de 0,02 à 0,05% m/v d'éosine disodique,

- de 50 à 80 % v/v d'éthanol, d'isopropanol ou d'un mélange éthanol/isopropanol, et
- de 20 à 50 % v/v d'eau.

Selon un mode de réalisation particulier, la composition liquide comprend, voire est exclusivement constituée de :

- 0,5% m/v de digluconate de chlorhexidine;
- de 0,02 à 0,05% m/v d'éosine disodique ;
- de 65 à 85% v/v, de préférence 70% v/v, d'éthanol, d'isopropanol ou d'un mélange éthanol/isopropanol et de 25 à 35% v/v, de préférence, 30% v/v, d'eau.

Dans les compositions comprenant de l'éosine disodique, le ratio molaire digluconate de chlorhexidine/éosine disodique sera de préférence dans la gamme allant de 7 à 33, et préférentiellement dans la gamme allant de 7 à 17.

Dans les compositions selon l'invention, les pourcentages m/v ou v/v sont exprimés par rapport au volume total de la composition. Les % préférés mentionnés pour chacun des composants seront, de préférence, utilisés en combinaison. En l'absence de composés liquides additionnels, la somme des % v/v donnés pour l'eau et le ou les alcools, sera égale à 100%.

L'eau utilisée dans les compositions selon l'invention sera, de préférence de l'eau purifiée, notamment au sens de la pharmacopée européenne.

Les compositions selon l'invention présentent, notamment, un pH dans la gamme allant de 6 à 7, et notamment proche de 7 ($7\pm10\%$).

Avantageusement, contrairement aux solutions proposées dans le document WO 2007/130981, les compositions selon l'invention ne comprennent pas de colorants cationiques et ne comprennent pas d'excipients cationiques, voire ne comprennent pas du tout de composés organiques cationiques autres que la chlorhexidine.

Par « composé organique », on entend un composé comprenant au moins un atome de carbone et au moins un atome d'hydrogène. Les composés organiques cationiques comprennent les colorants cationiques, les excipients cationiques et les détergents cationiques.

Un colorant cationique comprendra notamment au moins un groupement ammonium. De manière avantageuse, l'exclusion s'entendra exclusivement des colorants cationiques comprenant au moins un groupe ammonium, voire exclusivement des colorants cationiques spécifiquement cités, à savoir choisis parmi le cristal violet, le pur bleu victoria BO, le vert de méthyle, le vert de malachite, l'acriflavine, l'azure C et le marron bismarck.

Un excipient cationique, également nommé détergent cationique, comprendra notamment au moins un groupement ammonium. De manière avantageuse, l'exclusion s'entendra exclusivement des excipients cationiques comprenant au moins un groupe ammonium, voire exclusivement des excipients cationiques ou détergents cationiques spécifiquement cités, à savoir choisis parmi le chlorure de cétylpyridinium, le bromure d'hexadécylméthyle ammonium, le chlorure de benzéthonium et le chlorure de benzalkonium, le chlorure de méthylbenzéthonium, le chlorure de cétalkonium, le cétrimonium, le cétrimide, le chlorure de dofanium, le bromure de tétraéthylammonium, le chlorure de didécyldiméthylammonium et le bromure de domiphène.

L'invention a également pour objet les compositions selon l'invention pour leur utilisation en tant que médicament, et notamment en tant qu'antiseptique par application cutanée sur la peau humaine ou animale. L'application sera, de préférence, réalisée sur une zone du sujet devant subir une opération chirurgicale. De manière avantageuse, l'application d'une composition selon l'invention entraîne une coloration de la peau humaine visible sous scialytique et/ou sous lampe UV.

L'invention concerne également l'utilisation d'une composition selon l'invention pour l'antisepsie d'instruments médicaux ou chirurgicaux.

Les compositions selon l'invention présentent les avantages suivants :

- elles sont directement prêtes à l'emploi ;
- elles ne nécessitent pas une application avec un applicateur qui mélange extemporanément le colorant anionique et la chlorhexidine et sont donc faciles à mettre en œuvre ;
- elles présentent une excellente stabilité, avec absence de formation de précipité ;
- elles sont dépourvues de colorant anionique qui affecterait les qualités antiseptiques de la chlorhexidine ;
- elles ne présentent qu'un très faible risque d'hypersensibilité et sont moins nocives pour la santé que les compositions colorées de l'art antérieur ;
- elles permettent de marquer d'une couleur rouge la peau, cette couleur étant visible sous le scialytique ;
- elles permettent de délimiter la zone aseptisée de la peau en vue d'une intervention chirurgicale visible sous le scialytique ou sous une lampe UV, et ce, quel que soit la couleur de la peau ;
- elles peuvent être considérées comme une alternative pour les patients allergiques aux antiseptiques iodés la

Bétadine®.

Les compositions selon l'invention peuvent être qualifiées de stables. Par « stable », on entend notamment que la composition ne comprend pas de précipité visible à l'œil nu, notamment au bout d'un mois, de préférence au bout de 3 mois et plus préférentiellement, au bout de 6 mois de conservation. Plus particulièrement, une composition est stable, si sa concentration en digluconate de chlorhexidine ne varie pas substantiellement et/ou sa concentration en éthanol ne varie pas substantiellement, dans le temps, lorsque la composition est conservée à 25°C et à l'abri de la lumière. Il est considéré que la concentration en digluconate de chlorhexidine d'une composition ne varie pas substantiellement dans le temps, si sa concentration en digluconate de chlorhexidine après au moins 3 mois de conservation à 25°C et sous 60% d'humidité relative a varié de moins de 10%, de préférence si sa concentration en digluconate de chlorhexidine après au moins 6 mois de conservation à 25°C et sous 60% d'humidité relative a varié de moins de 10%, et de manière encore plus préférée si sa concentration en digluconate de chlorhexidine après au moins 12 mois de conservation à 25°C et sous 60% d'humidité relative a varié de moins de 10%. De même, il est considéré que la concentration en éthanol d'une composition ne varie pas substantiellement dans le temps, si sa concentration en éthanol après au moins 3 mois de conservation à 25°C et sous 60% d'humidité relative a varié de moins de 10%, de préférence si sa concentration en éthanol après au moins 6 mois de conservation à 25°C et sous 60% d'humidité relative a varié de moins de 10%, et de manière encore plus préférée si sa concentration en éthanol après au moins 12 mois de conservation à 25°C et sous 60% d'humidité relative a varié de moins de 10%. Les propriétés de stabilité des compositions de l'invention peuvent être déterminées conformément aux méthodes fournies dans les exemples.

La présente invention a également pour objet un procédé de fabrication d'une composition selon l'invention, comprenant les étapes suivantes :

(a) on dispose d'une première solution S1 ou on prépare une première solution S1 comprenant le digluconate de chlorhexidine et au moins un alcool choisi parmi l'éthanol, le n-propanol et l'isopropanol,
(b) on dispose d'une seconde solution S2 dans l'eau, ou on prépare une seconde solution S2 dans l'eau, d'au moins un sel pharmaceutiquement acceptable du colorant de formule (I) :

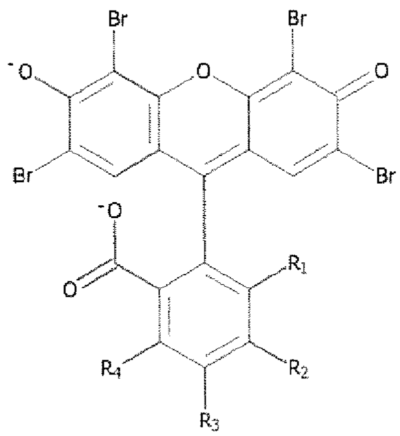

(I)

avec $R_1$, $R_2$, $R_3$, $R_4$, identiques ou différents qui représentent indépendamment un atome d'hydrogène ou un atome de chlore avec, de préférence, $R_1$, $R_2$, $R_3$, $R_4$ qui sont tous identiques, et représentent un atome d'hydrogène,
(c) on mélange la seconde solution S2 avec la première solution S1, de préférence par ajout de la seconde solution S2 à la première solution S1.

Ce procédé présente l'avantage d'être plus simple que les procédés de l'art antérieur. Il ne nécessite pas d'étape de filtration pour obtenir une composition stable.

La préparation des solutions S1 et S2 et le mélange de l'étape (c) seront réalisés sous une agitation appropriée, mécanique ou magnétique notamment.

De manière préférée, la première solution S1 est préparée en deux étapes (a1) et (a2), la première étape (a1) consistant à mélanger une solution de digluconate de chlorhexidine (20%) prête à l'emploi avec une solution d'alcool à 96%, et la seconde étape (a2) consistant en la dilution avec de l'eau de la solution obtenue à l'étape (a1), le facteur de dilution appartenant, de préférence, à une gamme allant de 1/2 à 1.

Avantageusement, l'étape (c) consiste en l'ajout d'une quantité suffisante de la solution S2 dans la première solution S1, permettant d'obtenir au final dans la composition un ratio molaire [digluconate de chlorhexidine]/[sel pharmaceutiquement acceptable du colorant de formule (I)] appartenant à la gamme allant de 7 à 33, de préférence à la gamme allant de 7 à 17.

Avantageusement, les étapes (a), (b), (c) s'effectuent à une température dans la gamme allant de 10 et 40°C, notamment à température ambiante (22°C).

De par leurs propriétés, les compositions de l'invention peuvent notamment être utilisées comme désinfectant, antiseptique de la peau, des mains, des plaies, des dispositifs médicaux et des instruments médicaux, notamment des instruments de chirurgie. Elles pourront être utilisées pour un marquage de la peau, ledit marquage étant visible sous scialytique (système d'éclairage utilisé dans les blocs opératoires) ou sous une lampe UV. L'application sur la peau d'un sujet de la composition peut se faire par toute technique connue de l'homme du métier, notamment par une application en badigeonnant la zone de la peau, ou bien par une application en tamponnant l'ensemble de la zone désirée.

Les compositions de l'invention contiennent des composants qui sont appropriés pour une utilisation au contact avec la peau. Ils sont sans toxicité, sans incompatibilité, sans instabilité, et sans réaction indésirable avec la peau. Les compositions selon l'invention pourront être conservées dans tout contenant approprié, permettant notamment une conservation à l'abri de la lumière. On privilégiera des contenants opaques, tels que des flacons en polyéthylène haute densité.

Les exemples ci-après permettent d'illustrer l'invention mais n'ont aucun caractère limitatif.

## Exemples

Les matières premières utilisées dans les exemples qui suivent sont listées ci-après :

- Solution aqueuse de digluconate de chlorhexidine à 20%
- Ethanol 96% (v/v)
- Eau purifiée
- Solution aqueuse d'éosine disodique 2%
- Jaune orangé
- Erythrosine
- Crystal violet
- Vert Méthyle

Les matières premières ont été utilisées telles que reçues des fabricants, sans purification supplémentaire.

Deux formulations de solutions alcooliques de digluconate de chlorhexidine colorées par de l'éosine disodique ont été préparées.

**Formulation 1 : Solution alcoolique à 71% v/v de digluconate de chlorhexidine 0,5% m/v et d'éosine disodique 0,02% m/v**

| | |
|---|---|
| Solution aqueuse de digluconate de chlorhexidine à 20% (m/v) | 3,14 ml |
| Quantité correspondant au digluconate de chlorhexidine | 0,63 g |
| Ethanol à 96 pour cent (v/v) | 93,75 ml |
| Eau purifiée | 28,11 ml |
| Solution aqueuse d'éosine disodique 2% (m/v) | 1,5 ml |

Pour 126,5 ml.

Titre alcoolique: **71% (v/v)** par rapport au volume total de la composition

Digluconate de chlorhexidine : 0,5 % (m/v)

Eosine disodique : 0,02% (m/v)

$$\text{Ratio molaire } \frac{\textit{Digluconate de chlorhexidine}}{\textit{Eosine disodique}} = 17$$

La Formulation 1 est préparée comme suit :

a) Une solution S1 d'éthanol à 71% (v/v) et de digluconate de chlorhexidine 0,5% (m/v) est obtenue:

**1.** En mélangeant 93,75 ml d'éthanol à 96° et 3,14 ml d'une solution aqueuse de digluconate de chlorhexidine à 20% (m/v) dans un bécher en verre. Une agitation manuelle ou magnétique permet la miscibilité des deux solutions et l'obtention d'un mélange limpide.
**2.** En ajoutant 28,11 ml d'eau purifiée au mélange précédent. Une agitation manuelle ou magnétique permet la miscibilité des deux solutions et l'obtention d'un mélange limpide.

b) Une solution aqueuse d'éosine disodique S2 à 2% (m/v) est préparée par simple dissolution d'éosine disodique dans de l'eau purifiée, dans un bécher en verre. Une agitation manuelle ou magnétique permet la dissolution de l'éosine disodique et l'obtention d'une solution colorée.
c) On mélange 1,5 ml de la solution S2 d'éosine disodique à 2% (m/v) à 125 ml de la solution S1 dans un bêcher en verre. Une agitation manuelle ou magnétique permet l'obtention d'une solution alcoolique 71% (v/v) de digluconate de chlorhexidine 0,5% (m/v) et d'éosine disodique 0,02% (m/v).

A titre de comparaison, si tous les constituants sont mélangés en même temps comme décrit à l'exemple B de la demande US 2008/0081020, une formulation instable est obtenue.

**Formulation 2 : Solution alcoolique 70 v/v de digluconate de chlorhexidine 0,5% m/v et d'éosine 0,05% m/v**

| | |
|---|---|
| Solution aqueuse de digluconate de chlorhexidine à 20% (m/v) | 3,14 ml |
| Quantité correspondant à digluconate de chlorhexidine | 0,63 g |
| Ethanol à 96 pour cent (v/v) | 93,75 ml |
| Eau purifiée | 28,11 ml |
| Solution aqueuse d'éosine disodique 2% (m/v) | 3 ml |

Pour 128 ml.

Titre alcoolique: 70 (v/v) ) par rapport au volume total de la composition.
Digluconate de chlorhexidine : 0,5 % (m/v)
Eosine disodique : 0,05% (m/v)

$$\text{Ratio molaire } \frac{\textit{Digluconate de chlorhexidine}}{\textit{Eosine disodique}} = 7$$

La Formulation 2 est préparée comme suit :

**a)** Une solution S1 identique à celle de la formulation 1 est préparée,
**b)** Une solution S2 d'éosine disodique identique à celle de la formulation 1 est préparée.
**c)** On mélange 3 ml de la solution S2 d'éosine disodique à 2% (m/v) à 125 ml de la solution S1 dans un bécher en verre. Une agitation manuelle ou magnétique permet l'obtention d'une solution alcoolique 70 (v/v) de digluconate de chlorhexidine 0,5% (m/v) et d'éosine disodique 0,05% (m/v).

1- Test du pouvoir colorant

Le pouvoir colorant de diverses compositions de digluconate de chlorhexidine contenant des colorants différents (éosine disodique, érythrosine, crystal violet et vert de méthyle) a été déterminé sur peau blanche (type caucasien) et noire en utilisant le système LAB à l'aide d'une sonde colorimétrique Skin-colorimeter® CL 400, CK Electronics colour measurement probe and C+K Multi Probe Adapter System MPA Courage Khazaka Electronic GmbH, Cologne, Allemagne).

***L*** représente la luminosité avec des valeurs de 0 à 100, ***a*** les couleurs du vert au rouge avec des valeurs de -128 à +128, et ***b*** les couleurs du bleu au jaune vert avec des valeurs de -128 à +128.

Les compositions avec d'autres colorants que l'éosine disodique ont été préparées selon le protocole décrit au paragraphe 1 et selon les quantités exprimées en % m/v présentées dans le **Tableau I** ci-après.

Pour chaque essai, un volume de 10 μL/cm$^2$ de composition a été appliqué sur la peau jusqu'à séchage complet.

Les résultats sont exprimés en pourcentage et présentés dans les **Tableaux I** et **II.** Le pouvoir colorant ΔE, $\Delta E = \sqrt{\Delta L^2 + \Delta a^2 + \Delta b^2}),$ est sans unité.

**Tableau I**

| **Colorants** | **($L_{colorant}$-$L_{peau}$)/$L_{peau}$ *100** | **($a_{colorant}$-$a_{peau}$)/$a_{peau}$** * 100 | **($b_{colorant}$-$b_{peau}$)/$b_{peau}$ *100** | **ΔE** |
|---|---|---|---|---|
| Eosine 0,02% (peau blanche) | -9,34% | 206,41% | -45,77% | 17,74 |
| Eosine 0,02% (peau noire) | 4,98% | 49,38% | -10,06% | 3,35 |
| Erythrosine 0,02% (peau blanche) | -3,01% | 142,11% | -81,33% | 12,59 |
| Erythrosine 0,02% (peau noire) | 5,50% | 4,74% | -24,61% | 4,78 |
| Crystal violet 0,02% | -13,61% | -48,09% | -115,44% | 14,06 |
| Vert de méthyle 0,02% | -13,81% | -128,69% | -42,18% | 14,30 |

**Tableau II**

| **Colorants** | **($L_{colorant}$-$L_{peau}$)/$L_{peau}$ *100** | **($a_{colorant}$-$a_{peau}$)/$a_{peau}$ * 100** | **($b_{colorant}$-$b_{peau}$)/$b_{peau}$ *100** | **ΔE** |
|---|---|---|---|---|
| Eosine 0,05% (peau blanche) | -11,91% | 372,48% | -14,82% | 24,26 |
| Eosine 0,05% (peau noire) | -0,67% | 68,95% | -1,37% | 3,74 |
| Erythrosine 0,05% (peau blanche) | -6,09% | 178,39% | -95,52% | 14,81 |
| Erythrosine 0,05% (peau noire) | -0,98% | 58,48% | -11,84% | 3,26 |
| Crystal violet 0,05% | -15,36% | -61,25% | -173,88% | 17,76 |
| Vert de méthyle 0,05% | -3,95% | -118,16% | -44,02% | 10,65 |

Ce test montre que l'éosine disodique possède le pouvoir colorant le plus intense, dans les tons rouges, visible sur peau blanche et peau noire.

**3.** Test de l'activité antiseptique

Afin d'observer une éventuelle inactivation ou un antagonisme de l'éosine disodique sur l'effet bactéricide et fongicide du digluconate de chlorhexidine 0,5%, des mesures de l'activité antimicrobienne des compositions comprenant 0,01 ; 0,02 ou 0,05% m/v d'éosine disodique ont été réalisées selon les normes en vigueur.

Une composition est considérée comme bactéricide vis-à-vis d'un microorganisme, lorsque celle-ci est capable d'entraîner une réduction logarithmique décimale du nombre de cellules bactériennes viables d'au moins 5 log, en d'autres termes, le taux de réduction du nombre de colonies viables doit être supérieur à 5.

Une composition est considérée comme fongicide et levuricide vis-à-vis d'un microorganisme, lorsque celle-ci est capable d'entraîner une réduction logarithmique décimale du nombre de cellules fongiques viables d'au moins 4 log, en d'autres termes, le taux de réduction du nombre de colonies viables doit être supérieur à 4.

Plus précisément, l'activité antibactérienne a été déterminée comme ci-dessous.

La conservation des souches bactériennes et la préparation des cultures de travail ont été faites selon la norme NF T-90-461.

La mesure de l'activité bactéricide des compositions à tester a été réalisée conformément à la norme NF EN 1040 avec deux souches bactériennes obligatoires: *Staphylococcus aureus* et *Pseudomonas aeruginosa* et conformément à la norme NF T 72-150 avec deux souches bactériennes additionnelles : *Enterococcus faecium* et *Mycobacterium smegmatis.* Les tests ont été effectués à 32°C pendant 5mn.

Les résultats d'activité antibactérienne de solutions alcooliques (70%) de digluconate de chlorhexidine (0,5%) en présence d'éosine disodique (0,01%, 0,02% ou 0,05%) sont présentés dans le Tableau **III.** Les rapports molaires de digluconate de chlorhexidine / éosine disodique sont indiqués entre crochets.

**Tableau III**

| Eosine (%) | Taux de réduction du nombre de colonies viables | | | |
|---|---|---|---|---|
| | *Enterococcus faecium CIP 5 855* | *Staphylococcus aureus CIP 53 154* | *Mycobacterium smegmatis CIP 7 326* | *Pseudomonas aeruginosa CIP A22* |
| 0,01 [33] | 7,14 | 7,44 | 7,16 | 7,25 |
| 0,02 [17] | 7,14 | 7,44 | 7,16 | 7,25 |
| 0,05 [7] | 7,14 | 5,28 | 7,16 | 7,25 |

L'activité antifongique et levuricide des compositions à tester a été déterminée selon la norme NF EN 1275 en utilisant 2 souches fongiques obligatoires : *Aspergillus brasiliensis et Candida albicans,* le test s'effectuant à 20°C et pendant une durée de 15 min. Les résultats d'activité antifongiques de solutions alcooliques (70%) de digluconate de chlorhexidine (0,5%) en présence d'éosine disodique (0,01%, 0,02% ou 0,05%) sont présentés dans le **Tableau IV.** Les rapports molaires de digluconate de chlorhexidine / éosine disodique sont indiqués entre crochets.

**Tableau IV**

| Eosine (%) | Taux de réduction du nombre de colonies viables | |
|---|---|---|
| | *Aspergillus brasiliensis ATCC 16 404* | *Candida albicans ATCC 10 231* |
| 0,01 [33] | 6,62 | 6,51 |
| 0,02 [17] | 6,62 | 6,51 |
| 0,05 [7] | 6,62 | 6,51 |

Ces essais mettent en évidence que les différentes concentrations d'éosine disodique ne modifient pas l'activité bactéricide, fongicide et levuricide d'une composition alcoolique comprenant un mélange de digluconate de chlorhexidine et d'éosine disodique.

**4.** Etude de stabilité

Trois tests de stabilités ont été réalisés l'un immédiatement après la fabrication de la composition, l'un dans des conditions normales de conservation, et le dernier dans des conditions de vieillissement accéléré.

Une analyse microbiologique (test bioburden) des compositions a été également réalisée incluant le dénombrement de la flore aérobie, des moisissures et des levures totales, de *Pseudomonas aeruginosa* et de *Staphylococcus aureus*. Cette analyse a permis d'attester de l'absence de contamination par des germes lors de la fabrication des compositions.

*4.1 Etude de la stabilité de la composition après sa fabrication*

Le test de stabilité après la fabrication de la composition consiste en un examen visuel de la composition, afin de déterminer la présence ou non d'un précipité.

Deux compositions ont été testées : la formulation 1 comprenant l'éosine disodique à 0,02% m/v et une composition similaire dans laquelle l'éosine disodique a été remplacée par un autre colorant anionique : le jaune orangé (référence).

L'ajout du colorant anionique jaune orangé dans une solution d'éthanol 70% v/v de digluconate de chlorhexidine 0,5% m/v induit une instabilité avec la formation de précipités incompatibles avec une administration cutanée. La formation de précipité est visible dans ce cas.

Bien que l'éosine soit également un colorant anionique, la formulation 1 comprenant 0,02% m/v d'éosine disodique est stable. En effet, aucune formation de précipité n'a été observée pour les formulations 1 et 2.

Toutefois, une formulation comprenant 0,11% m/v d'éosine disodique, et un ratio molaire digluconate de chlorhexidine / éosine disodique égal à 3 (référence) a été préparée de la même façon. Cette composition présentait une instabilité.

Dans ce cas, un précipité a été observé pour cette composition comprenant 0,11% m/v d'éosine disodique : des agrégats ou agglomérats de couleur rouge ont sédimenté au fond du flacon instantanément dès la fabrication de la composition, conduisant à un dépôt.

*4.2 Etude de la stabilité de la composition dans des conditions normales de conservation et dans des conditions de vieillissement accéléré*

Une étude de stabilité a été conduite pendant 12 mois pour les formulations 1 et 2 selon les recommandations de *l'International Conférence on Harmonization* dans deux conditions de conservation :

- à 25°C ± 2°C et 60% ± 5% en humidité relative (conditions normales de conservation) et
- à 40°C ± 2°C, 75% ± 5% en humidité relative (conditions de vieillissement accéléré).

Les compositions ont été conservées dans des flacons opaques en polyéthylène haute densité.

La première analyse a consisté à vérifier visuellement l'apparence de la composition et confirmer l'absence de précipité à l'issue de la période de stockage.

Puis, un dosage du digluconate de chlorhexidine a été réalisé par chromatographie en phase liquide à ultra-haute performance (UHPLC) à l'issue de chaque période de stockage. Cette méthode séparative permet de détecter d'éventuels produits de dégradation apparaissant au cours de l'étude de stabilité.

L'éthanol a été également dosé à l'issue de chaque période de stockage. Ce dosage a été effectué par chromatographie en phase gazeuse (CPG).

Les résultats de stabilité de la formulation 1 dans des conditions de conservation à 25°C et 60% d'humidité relative sont présentés dans le **Tableau V.**

## Tableau V

| Paramètres organoleptiques et physicochimiques | Période de stockage | | | | | |
|---|---|---|---|---|---|---|
| | Initial | 15 jours | 1 mois | 2 mois | 3 mois | 6 mois |
| Aspect | limpide | limpide | limpide | limpide | limpide | limpide |
| Couleur | Rouge | Rouge | Rouge | Rouge | Rouge | Rouge |
| Concentration % m/v de digluconate de chlorhexidine [a] (concentration théorique : [0,45 – 0,55] % ± 3 %) | 0,49 | 0,48 | 0,46 | 0,49 | 0,50 | 0,49 |
| Concentration éthanol % v/v [b] (concentration théorique : [63,9 – 78,1] % ± 5 %) | 69,0 | 76,6 | 81,5* | 66,8 | 70,4 | 81,5* |
| Conformité | Oui | Oui | Oui | Oui | Oui | Oui |

* Non significativement différent de la valeur limite de 78.1%

[a] : UHPLC

[b] : CPG

**Tableau V (suite)**

| Paramètres organoleptiques et physicochimiques | Période de stockage | | |
|---|---|---|---|
| | 1 an | 18 mois | 24 mois |
| Aspect | limpide | limpide | limpide |
| Couleur | Rouge | Rouge | Rouge |
| Concentration % m/v de digluconate de chlorhexidine [a] (concentration théorique : [0,45 - 0,55] % ± 3 %) | 0,46 | 0,46 | 0,45 |
| Concentration éthanol % v/v [b] (concentration théorique : [63,9 - 78,1] % ± 5 %) | 70,3 | 74,3 | 63,6* |
| Conformité | Oui | Oui | Oui |

* Non significativement différent de la valeur limite de 78.1%
[a] : UHPLC
[b] : CPG

Les résultats de stabilité de la formulation 1 dans des conditions de conservation à 40°C et 75% d'humidité relative sont présentés dans le **Tableau VI.**

**Tableau VI**

| Paramètres organoleptiques et physicochimiques | Période de stockage | | | | | | |
|---|---|---|---|---|---|---|---|
| | Initial | 15 jours | 1 mois | 2 mois | 3 mois | 6 mois | 1 an |
| Aspect | limpide | limpide | limpide | limpide | limpide | limpide | limpide |
| Couleur | rouge | rouge | rouge | rouge | rouge | rouge | rouge |
| Concentration % m/v de digluconate de chlorhexidine[a] (concentration théorique : [0,45 - 0,55] % ± 3 %) | 0,49 | 0,46 | 0,45 | 0,46 | 0,48 | 0,45 | 0,21 |
| Concentration éthanol % v/v [b] (concentration théorique : [63,9 - 78,1] % ± 5 %) | 69,0 | 79,1* | 74,2 | 68,6 | 72,0 | 74,3 | 70,5 |
| Conformité | Oui | Oui | Oui | Oui | Oui | Oui | NON |

* Non significativement différent de la valeur limite de 78.1%
[a] : UHPLC
[b] : CPG

Les résultats des Tableaux **V** et **VI** montrent que la formulation 1 est stable jusqu'à 12 mois pour des conditions de conservation à 25° et 60% d'humidité relative et pour des conditions de conservation à 40°C et 75 % d'humidité relative.

Les résultats de stabilité de la formulation 2 dans des conditions de conservation à 25°C et 60% d'humidité relative sont présentés dans le **Tableau VII.**

**Tableau VII**

| | **Période de stockage** | | | | |
|---|---|---|---|---|---|
| Paramètres organoleptiques et physicochimiques | **Initial** | **15 jours** | **1 mois** | **2 mois** | **3 mois** |
| Aspect | Limpide | Limpide | Limpide | Limpide | Limpide |
| Couleur | Rouge | Rouge | Rouge | Rouge | Rouge |
| Concentration % m/v de digluconate de chlorhexidine [a] (concentration théorique : [0,45 - 0,55] % $\pm$ 3 %) | 0,47 | 0,50 | 0,48 | 0,49 | 0,48 |
| Concentration éthanol % v/v [b] (concentration théorique : [63,9 - 78,1] % $\pm$ 5 %) | 67,5 | 69,1 | 69,1 | 67,2 | 70,9 |
| Conformité | Oui | Oui | Oui | Oui | Oui |

[a] : UHPLC
[b] : CPG

Les résultats de stabilité de la formulation 2 dans des conditions de conservation à 40°C et 75% d'humidité relative sont présentés dans le **Tableau VIII.**

**Tableau VIII**

| | **Période de stockage** | | | | |
|---|---|---|---|---|---|
| Paramètres organoleptiques et physicochimiques | **Initial** | **15 jours** | **1 mois** | **2 mois** | **3 mois** |
| Aspect | Limpide | Limpide | Limpide | Limpide | Limpide |
| Couleur | Rouge | Rouge | Rouge | Rouge | Rouge |
| Concentration % m/v de digluconate de chlorhexidine [a] (concentration théorique : [0,45-0,55] % $\pm$ 3 %) | 0,47 | 0,46 | 0,47 | 0,46 | 0,44* |
| Concentration éthanol % v/v [b] (concentration théorique : [63,9 - 78,1] % $\pm$ 5 %) | 67,5 | 71,1 | 71,1 | 67,5 | 75,3 |
| Conformité | Oui | Oui | Oui | Oui | Oui |

* Non significativement différent de la valeur limite de 0,45%
[a] : UHPLC
[b] : CPG

Les résultats des Tableaux **VII** et **VIII** montrent que la formulation 2 est stable jusqu'à 3 mois au moins, pour des conditions de conservation à 25° et 60% d'humidité relative et pour des conditions de conservation à 40°C et 75 % d'humidité relative.

## Revendications

**1.** Composition liquide stable comprenant :

- du digluconate de chlorhexidine,
- un colorant de formule (I) sous la forme d'un sel pharmaceutiquement acceptable :

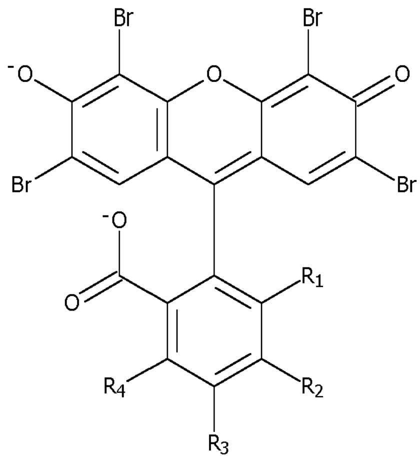

(I)

dans laquelle $R_1$, $R_2$, $R_3$, $R_4$, identiques ou différents, représentent chacun indépendamment un atome d'hydrogène ou un atome de chlore,
- de l'eau,
- et au moins un alcool choisi parmi l'éthanol, le n-propanol et l'isopropanol, ladite composition comprenant de 0,4 à 2,5% m/v, par rapport au volume total de la composition, de digluconate de chlorhexidine et de 0,01 à 0,06% m/v, par rapport au volume total de la composition, d'un sel pharmaceutiquement acceptable du colorant de formule (I).

**2.** Composition selon la revendication 1, **caractérisée en ce que** le ratio molaire [digluconate de chlorhexidine]/[colorant de formule (I) sous la forme d'un sel pharmaceutiquement acceptable] appartient à la gamme allant de 6 à 197, de préférence à la gamme allant de 7 à 79.

**3.** Composition selon la revendication 1 ou 2, **caractérisée en ce qu'**elle comprend de 0,5 à 2 % m/v de digluconate de chlorhexidine et de 0,02 à 0,05% m/v d'un sel pharmaceutiquement acceptable du colorant de formule (I), par rapport au volume total de la composition.

**4.** Composition selon l'une quelconque des revendications 1 à 3, **caractérisée en ce que** le ratio molaire [digluconate de chlorhexidine]/[colorant de formule (I) sous la forme d'un sel pharmaceutiquement acceptable] appartient à la gamme allant de 7 à 33, et préférentiellement à la gamme allant de 7 à 17.

**5.** Composition selon l'une quelconque des revendications 1 à 4, **caractérisée en ce que**, dans le colorant de formule (I), $R_1$, $R_2$, $R_3$ et $R_4$ sont tous identiques et représentent un atome d'hydrogène.

**6.** Composition selon l'une quelconque des revendications 1 à 5, **caractérisée en ce que** le colorant de formule (I) se présente sous la forme de son sel disodique.

**7.** Composition selon l'une quelconque des revendications 1 à 6, **caractérisée en ce qu'**elle comprend de 5 à 50 % v/v d'eau et de 50 à 95 % v/v d'alcool choisi parmi l'éthanol, le n-propanol et l'isopropanol et leurs mélanges, de préférence de 20 à 50 % v/v d'eau et de 50 à 80 % v/v d'alcool choisi parmi l'éthanol, le n-propanol et l'isopropanol et leurs mélanges, les % v/v étant exprimés par rapport au volume total de la composition.

**8.** Composition selon l'une quelconque des revendications 1 à 7, **caractérisée en ce qu'**elle comprend :

- de 0,5 à 2% m/v de digluconate de chlorhexidine;
- de 0,02 à 0,05% m/v de colorant de formule (I) sous la forme de son sel disodique avec $R_1$, $R_2$, $R_3$ et $R_4$ tous identiques et représentant un atome d'hydrogène;
- de 20 à 50 % v/v d'eau,
- de 50 à 80% v/v d'éthanol ou d'isopropanol ou d'un mélange éthanol/isopropanol,

les pourcentages m/v ou v/v étant exprimés par rapport au volume total de la composition.

**9.** Composition selon l'une quelconque des revendications 1 à 8, **caractérisée en ce que** les compositions ne comprennent pas de colorants cationiques, ne comprennent pas d'excipients cationiques, voire ne comprennent pas de composés organiques cationiques autres que la chlorhexidine.

**10.** Composition selon l'une quelconque des revendications 1 à 9, pour son utilisation en tant que médicament, et notamment en tant qu'antiseptique par application cutanée sur la peau humaine ou animale.

**11.** Composition selon la revendication 10, **caractérisée en ce que** l'application est réalisée sur une zone du sujet devant subir une opération chirurgicale.

**12.** Composition selon la revendication 10 ou 11, **caractérisée en ce que** l'application entraîne une coloration de la peau visible sous scialytique et/ou sous lampe UV.

**13.** Procédé de fabrication d'une composition selon l'une quelconque des revendications 1 à 9, **caractérisé en ce qu'**il comprend les étapes suivantes:

(a) on dispose d'une première solution S1 ou on prépare une première solution S1 comprenant le digluconate de chlorhexidine et au moins un alcool choisi parmi l'éthanol, le n-propanol et l'isopropanol,
(b) on dispose d'une seconde solution S2 dans l'eau, ou on prépare une seconde solution S2 dans l'eau, d'au moins un sel pharmaceutiquement acceptable du colorant de formule (I),

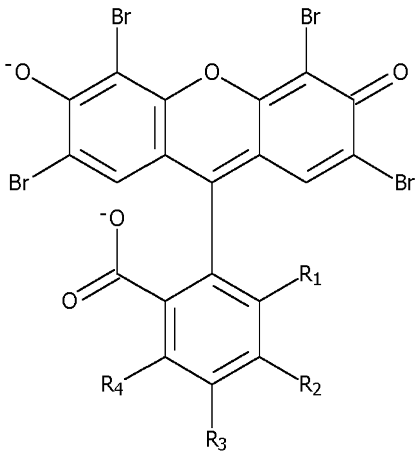

(I)

avec $R_1$, $R_2$, $R_3$, $R_4$, identiques ou différents qui représentent indépendamment un atome d'hydrogène ou un atome de chlore avec, de préférence, $R_1$, $R_2$, $R_3$, $R_4$ qui sont tous identiques, et représentent un atome d'hydrogène,
(c) on mélange la seconde solution S2 avec la première solution S1, de préférence par ajout de la seconde solution S2 à la première solution S1.

**14.** Procédé selon la revendication 13, **caractérisé en ce que** l'étape (c) consiste en l'ajout d'une quantité suffisante de la solution S2 dans la première solution S1, permettant d'obtenir au final dans la composition un ratio molaire [digluconate de chlorhexidine]/[sel pharmaceutiquement acceptable du colorant de formule (I)] appartenant à la gamme allant de 7 à 33, de préférence à la gamme allant de 7 à 17.

**15.** Utilisation d'une composition selon l'une quelconque des revendications 1 à 9 pour l'antisepsie d'instruments médicaux ou chirurgicaux.

## Patentansprüche

**1.** Stabile flüssige Zusammensetzung umfassend:

- Chlorhexidindigluconat,
- einen Farbstoff der Formel (I) in Form eines pharmazeutisch verträglichen Salzes:

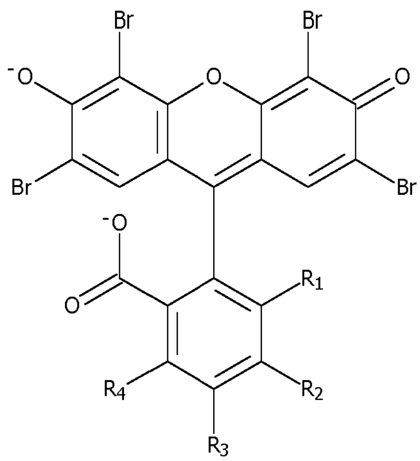

(I)

in der $R_1$, $R_2$, $R_3$, $R_4$ identisch oder verschieden sind und jeweils unabhängig voneinander ein Wasserstoffatom oder ein Chloratom darstellen,

- Wasser,
- und zumindest einen Alkohol, der unter Ethanol, n-Propanol und Isopropanol ausgewählt ist,

wobei die Zusammensetzung 0,4 bis 2,5 % m/v, bezogen auf das Gesamtvolumen der Zusammensetzung, des Chlorhexidindigluconats und des 0,01 bis 0,06 % m/v, bezogen auf das Gesamtvolumen der Zusammensetzung eines pharmazeutisch verträglichen Salzes des Farbstoffs der Formel (I) umfasst.

2. Zusammensetzung nach Anspruch 1, **dadurch gekennzeichnet, dass** das Molverhältnis [Chlorhexidindigluconat] /[Farbstoff der Formel (I) in Form eines pharmazeutisch verträglichen Salzes] im Bereich von 6 bis 197, vorzugsweise im Bereich von 7 bis 79 liegt.

3. Zusammensetzung nach Anspruch 1 oder 2, **dadurch gekennzeichnet, dass** sie 0,5 bis 2 % m/v Chlorhexidindigluconat und 0,02 bis 0,05 % m/v eines pharmazeutisch verträglichen Salzes des Farbstoffs der Formel (I), bezogen auf das Gesamtvolumen der Zusammensetzung, umfasst.

4. Zusammensetzung nach einem beliebigen der vorstehenden Ansprüche 1 bis 3, **dadurch gekennzeichnet, dass** das Molverhältnis [Chlorhexidindigluconat]/[Farbstoff der Formel (I) in Form eines pharmazeutisch verträglichen Salzes] im Bereich von 7 bis 33 und vorzugsweise im Bereich von 7 bis 17 liegt.

5. Zusammensetzung nach einem beliebigen der vorstehenden Ansprüche 1 bis 4, **dadurch gekennzeichnet, dass** im Farbstoff der Formel (I) $R_1$, $R_2$, $R_3$, $R_4$ alle identisch sind und ein Wasserstoffatom darstellen.

6. Zusammensetzung nach einem beliebigen der vorstehenden Ansprüche 1 bis 5, **dadurch gekennzeichnet, dass** der Farbstoff der Formel (I) die Form seines Dinatriumsalzes hat.

7. Zusammensetzung nach einem beliebigen der vorstehenden Ansprüche 1 bis 6, **dadurch gekennzeichnet, dass** sie 5 bis 50 % v/v Wasser und 50 bis 95 % v/v Alkohol, unter Ethanol, n-Propanol und Isopropanol und deren Mischungen ausgewählt, vorzugsweise 20 bis 50 % v/v Wasser und 50 bis 80 % v/v Alkohol, unter Ethanol, n-Propanol und Isopropanol und deren Mischungen ausgewählt, umfasst, wobei die v/v % bezogen auf das Gesamtvolumen der Zusammensetzung ausgedrückt sind.

8. Zusammensetzung nach einem beliebigen der vorstehenden Ansprüche 1 bis 7, **dadurch gekennzeichnet, dass** sie Folgendes umfasst:

- von 0,5 bis 2 % m/v Chlorhexidindigluconat;

- von 0,02 bis 0,05 % m/v Farbstoff der Formel (I) in Form seines Dinatriumsalzes mit $R_1$, $R_2$, $R_3$, $R_4$, die alle identisch sind und ein Wasserstoffatom darstellen;
- von 20 bis 50 % v/v Wasser,
- 50 bis 80 % v/v Ethanol oder Isopropanol oder eine Mischung aus Ethanol/Isopropanol,

wobei die m/v oder v/v Prozentsätze bezogen auf das Gesamtvolumen der Zusammensetzung ausgedrückt sind.

**9.** Zusammensetzung nach einem beliebigen der vorstehenden Ansprüche 1 bis 8, **dadurch gekennzeichnet, dass** die Zusammensetzungen keine kationischen Farbstoffe, keine kationischen Hilfsstoffe und auch keine kationischen organischen Verbindungen außer Chlorhexidin umfassen.

**10.** Zusammensetzung nach einem beliebigen der vorstehenden Ansprüche 1 bis 9 zur Verwendung als Arzneimittel, und insbesondere als Antiseptikum durch kutane Anwendung auf menschlicher oder tierischer Haut.

**11.** Zusammensetzung nach Anspruch 10, **dadurch gekennzeichnet, dass** die Anwendung auf einem Bereich des Probanden durchgeführt wird, der sich einem chirurgischen Eingriff unterziehen muss.

**12.** Zusammensetzung nach Anspruch 10 oder 11, **dadurch gekennzeichnet, dass** die Anwendung zu einer unter einer Operationsleuchte und/oder einer UV-Lampe sichtbaren Hautfärbung führt.

**13.** Verfahren zur Herstellung einer Zusammensetzung nach einem beliebigen der vorstehenden Ansprüche 1 bis 9, **dadurch gekennzeichnet, dass** es die folgenden Schritte umfasst:

(a) Eine erste Lösung S1 ist bereitgestellt, oder man bereitet eine erste Lösung S1 zu, die Chlorhexidindigluconat und zumindest einen Alkohol umfasst, der unter Ethanol, n-Propanol und Isopropanol ausgewählt ist,
(b) Eine zweite Lösung S2 in Wasser ist bereitgestellt, oder eine zweite Lösung S2 in Wasser von zumindest einem pharmazeutisch verträglichen Salz des Farbstoffs der Formel (I) wird zubereitet,

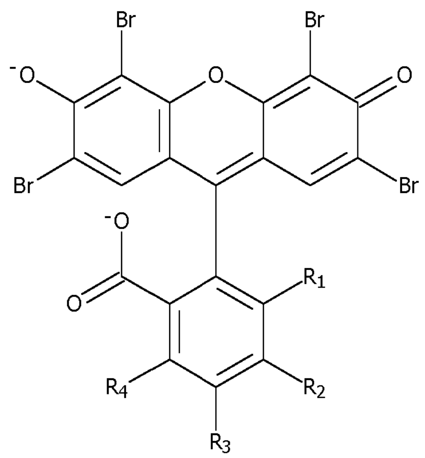

(I)

wobei $R_1$, $R_2$, $R_3$, $R_4$ identisch oder verschieden sind und unabhängig voneinander ein Wasserstoffatom oder ein Chloratom darstellen, bei welcher $R_1$, $R_2$, $R_3$, $R_4$ vorzugsweise alle identisch sind und ein Wasserstoffatom darstellen,
(c) die zweite Lösung S2 wird mit der ersten Lösung S1 vorzugsweise durch Hinzufügung der zweiten Lösung S2 zur ersten Lösung S1 gemischt.

**14.** Verfahren nach Anspruch 13, **dadurch gekennzeichnet, dass** der Schritt (c) in der Hinzufügung einer ausreichenden Menge der S2 Lösung zur ersten S1 Lösung besteht, was es ermöglicht, schließlich in der Zusammensetzung ein Molverhältnis [Chlorhexidindigluconat]/[pharmazeutisch verträgliches Salz des Farbstoffs der Formel (I)] zu erhalten, das im Bereich von 7 bis 33, vorzugsweise im Bereich von 7 bis 17 liegt.

15. Verwendung einer Zusammensetzung nach einem beliebigen der vorstehenden Ansprüche 1 bis 9 zur Antisepsis von medizinischen oder chirurgischen Instrumenten.

## Claims

1. A stable liquid composition comprising:

- chlorhexidine digluconate,
- a dye of formula (I) in the form of a pharmaceutically acceptable salt:

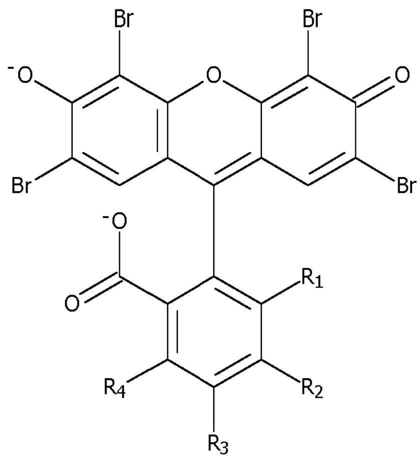

(I)

wherein $R_1$, $R_2$, $R_3$, $R_4$, identical or different, each independently represent a hydrogen atom or a chlorine atom,
- water,
- and at least one alcohol selected from ethanol, n-propanol and isopropanol,

said composition comprising from 0.4 to 2.5% w/v, in relation to the total volume of the composition, of chlorhexidine digluconate and from 0.01 to 0.06% w/v, in relation to the total volume of the composition, of a pharmaceutically acceptable salt of the dye of formula (I).

2. The composition according to claim 1, **characterized in that** the molar ratio [chlorhexidine digluconate]/[dye of formula (I) in the form of a pharmaceutically acceptable salt] belongs to the range from 6 to 197, preferably to the range from 7 to 79.

3. The composition according to claim 1 or 2, **characterized in that** it comprises from 0.5 to 2% w/v of chlorhexidine digluconate and from 0.02 to 0.05% w/v of a pharmaceutically acceptable salt of the dye of formula (I), in relation to the total volume of the composition.

4. The composition according to any one of claims 1 to 3, **characterized in that** the molar ratio [chlorhexidine digluconate]/[dye of formula (I) in the form of a pharmaceutically acceptable salt] belongs to the range from 7 to 33, and preferentially to the range from 7 to 17.

5. The composition according to any one of claims 1 to 4, **characterized in that**, in the dye of the formula (I), $R_1$, $R_2$, $R_3$ and $R_4$ are all identical and represent a hydrogen atom.

6. The composition according to any one of claims 1 to 5, **characterized in that** the dye of formula (I) is in the form of its disodium salt.

7. The composition according to any one of claims 1 to 6, **characterized in that** it comprises from 5 to 50% v/v of water and from 50 to 95% v/v of alcohol selected from ethanol, n-propanol and isopropanol and mixtures thereof, preferably from 20 to 50% v/v of water and from 50 to 80% v/v of alcohol selected from ethanol, n-propanol and isopropanol and mixtures thereof, the v/v% being expressed in relation to the total volume of the composition.

**8.** The composition according to any one of claims 1 to 7, **characterized in that** it comprises:

- from 0.5 to 2% w/v of chlorhexidine digluconate;
- from 0.02 to 0.05% w/v of dye of formula (I) in the form of its disodium salt with $R_1$, $R_2$, $R_3$ and $R_4$, all identical and representing a hydrogen atom;
- from 20 to 50% v/v of water,
- from 50 to 80% v/v of ethanol or isopropanol or an ethanol/isopropanol mixture,

the w/v or v/v percentages being expressed in relation to the total volume of the composition.

**9.** The composition according to any one of claims 1 to 8, **characterized in that** the compositions do not comprise cationic dyes, do not comprise cationic excipients, or even do not comprise cationic organic compounds other than chlorhexidine.

**10.** The composition according to any one of claims 1 to 9, for its use as a medicinal product, and in particular as an antiseptic by cutaneous application to human or animal skin.

**11.** The composition according to claim 10, **characterized in that** the application is carried out on an area of the subject having to undergo a surgical operation.

**12.** The composition according to claim 10 or 11, **characterized in that** the application causes a coloration of the skin visible under a surgical light and/or under a UV lamp.

**13.** A process for manufacturing a composition according to any one of claims 1 to 9, **characterized in that** it comprises the following steps:

(a) a first solution S1 is available or a first solution S1 is prepared comprising chlorhexidine digluconate and at least one alcohol selected from ethanol, n-propanol and isopropanol,
(b) a second solution S2 in water is available, or a second solution S2 in water is prepared, of at least one pharmaceutically acceptable salt of the dye of formula (I),

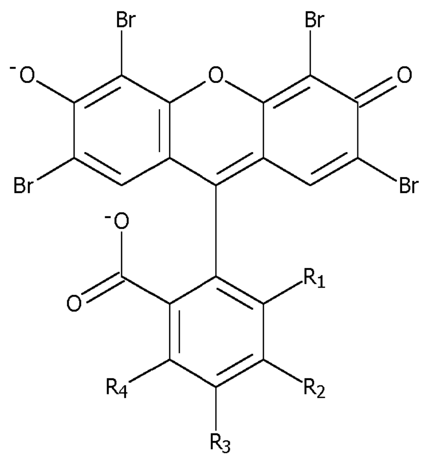

(I)

with $R_1$, $R_2$, $R_3$, $R_4$, identical or different, which independently represent a hydrogen atom or a chlorine atom with, preferably, $R_1$ $R_2$, $R_3$, $R_4$, which are all identical, and represent a hydrogen atom,
(c) the second solution S2 is mixed with the first solution S1, preferably by adding the second solution S2 to the first solution S1.

**14.** The process according to claim 13, **characterized in that** step (c) consists in adding a sufficient amount of the solution S2 to the first solution S1, so as to ultimately obtain in the composition a molar ratio [chlorhexidine digluconate] /[pharmaceutically acceptable salt of the dye of formula (I)] belonging to the range from 7 to 33, preferably to the

range from 7 to 17.

**15.** Use of a composition according to any one of claims 1 to 9 for the antisepsis of medical or surgical instruments.

## RÉFÉRENCES CITÉES DANS LA DESCRIPTION

*Cette liste de références citées par le demandeur vise uniquement à aider le lecteur et ne fait pas partie du document de brevet européen. Même si le plus grand soin a été accordé à sa conception, des erreurs ou des omissions ne peuvent être exclues et l'OEB décline toute responsabilité à cet égard.*

### Documents brevets cités dans la description

- US 6337357 B
- EP 2499913 A
- US 20080081020 A
- US 20030059379 A
- FR 2791890
- WO 2007130981 A

### Littérature non-brevet citée dans la description

- **BLECH, M. F. ; MARTIN, C. ; PICHON, M. et al.** Clinical and bacteriologic course of wounds as a function of various protocols of local antisepsis. *Revue de chirurgie orthopédique et réparatrice de l'appareil moteur,* 1989, vol. 76 (1), 55-61
- **CHOW, JASON ; NG, JONATHAN ; PUN, ALVIN.** Effects of food colouring added to 2% chlorhexidine gluconate and 70% alcohol for surgical site antisepsis. *Journal of perioperative practice,* 2013, vol. 23 (11), 255-257
- *CHEMICAL ABSTRACTS,* 18472-51-0
- *CHEMICAL ABSTRACTS,* 17372-87-1